Europäisches Patentamt

European Patent Office

Office européen des brevets

(11) Publication number: **0 182 689 B1**

# EUROPEAN PATENT SPECIFICATION

(45) Date of publication of patent specification: **27.12.91**  (51) Int. Cl.⁵: **A61M 29/02, A61B 17/22, A61B 17/38, A61M 25/10**

(21) Application number: **85402067.4**

(22) Date of filing: **24.10.85**

(54) Apparatus for angioplasty.

(30) Priority: **24.10.84 US 664156**

(43) Date of publication of application:
**28.05.86 Bulletin 86/22**

(45) Publication of the grant of the patent:
**27.12.91 Bulletin 91/52**

(84) Designated Contracting States:
**DE FR GB IT**

(56) References cited:
**WO-A-20/0768**
**WO-A-40/4879**
**FR-A-24 801 07**
**US-A- 4 449 528**

(73) Proprietor: **THE BETH ISRAEL HOSPITAL AS-
SOCIATION**
**330 Brookline Avenue**
**Boston, MA 02109(US)**

(72) Inventor: **Spears, J. Richard**
**400 Brookline Avenue, Apt. 17E**
**Boston Massachusetts 02215(US)**

(74) Representative: **Bassett, Richard Simon et al**
**ERIC POTTER & CLARKSON St. Mary's Court**
**St. Mary's Gate**
**Nottingham NG1 1LE(GB)**

Rank Xerox (UK) Business Services

## Description

FIELD OF INVENTION

This invention relates to an angioplasty device which enables the prevention of abrupt reclosure and restenosis after treatment.

BACKGROUND OF THE INVENTION

The mortality and morbidity from ischemic heart disease results primarily from atheromatous narrowings of the coronary arteries. Although various medical and surgical therapies may improve the quality of lifestyle of most patients with symptomatic coronary atherosclerosis, these therapies do not favorably change the underlying anatomy responsible for the coronary luminal narrowings and therefore do not prevent the occurrence of future cardiac events such as occurrence of worsening of signs and symptoms of myocardial ischemia, myocardial infarction and sudden death.

Percutaneous transluminal coronary angioplasty (PTCA) has recently been developed as an alternative method of treatment of coronary atherosclerosis. During cardiac catheterization an inflatable balloon is inserted in a coronary artery at the region of a coronary narrowing. Inflation of the balloon for 15-60 seconds results in expansion of the narrowed lumen or passageway. Because residual narrowing is usually present after the first balloon inflation, multiple inflations are routinely performed in an attempt to reduce the severity of the residual stenosis or tube narrowing. Despite these multiple inflations, a mild to moderately severe residual stenosis usually is present after successful PTCA.

One of the major problems with such treatment is that a flap of material occasionally is formed during the treatment which, after withdrawal of the instrumentation, falls back into the artery causing abrupt reclosure. This necessitates emergency coronary artery bypass surgery and thus PTCA is potentially dangerous and often provides only a temporary treatment of symptoms of obstructive coronary arterial atherosclerosis. The reason that the flap is formed is that upon balloon inflation the surrounding material is broken or fragmented which causes blood to enter the arterial wall between planes of dissection. This causes the arterial wall to swell acutely and either reduces the size of the channel or completely obliterates the channel resulting in a five percent incidence of abrupt reclosure.

Moreover with present PTCA procedures, postmortem pathologic studies show that disruption of the arterial wall and atheromatous plaque occurs following balloon inflation, including fracture of the plaque and separation of tissue layers, e.g., dissection. Angiographically a shaggy or hazy appearance of the coronary lumen is often seen and evidence for overt dissection is often apparent following successful PTCA. Disruption of the arterial wall temporarily increases the size of the coronary channel but predisposes to deposition of platelets and microthrombi which very likely contribute to the greater than 25% incidence of restenosis within three to six months following successful PTCA.

By way of further background, recent studies have been reported using lasers to perform vascular anastomoses so that end-to-end and end-to-side arterial and vein grafting can be achieved without sutures. The basic principle simply involves placing the free edges of a vessel and the graft in close proximity and heating these tissues with either an argon-ion, neodymium : YAG, or $CO_2$ laser. Crosslinking of collagen and other tissue proteins occurs and a coagulation is observed pathologically following the treatment. The tissue integrity is maintained, however, and the tensile strength of the "sutureless" anastomosis rivals that of anastomoses performed with sutures used in a conventional manner. Moreover, short and long term tissue healing appears to be better with the laser thermal fusion of tissues than with the suture technique.

WO-A-84/01 903 discloses an angiography catheter comprising, among others, an elongated tubular catheter body 14 and an annular balloon means 26 having an inflatable and deflatable dilatation portion 28 having, in turn, a mouth peripherally sealed to the distal end portion of said body 14. The dilatation portion 28 is inverted within the catheter body 14 and evertable therefrom. An interior, always-open lumen 42 of the catheter is defined in part by the balloon means 26; through this lumen 42, various substances or objects may be passed.

SUMMARY OF THE INVENTION

The present invention relates to an angioplasty device comprising a catheter suitable for insertion into an artery and having:
a tube defining a lumen;
an inflatable balloon secured to said tube for inflation from a remote source of fluid; and
means for inflating said balloon;
said balloon being configured so that said tube may be navigated through the artery when deflated and allow blood to flow; and
the means for inflating said balloon being configured so that they may inflate said balloon with fluid when said balloon is in an area of stenosis of the artery to apply pressure to the area of stenosis of said artery to widen the inner diameter of the artery at that area: characterized by

means for transmitting heat through the balloon.

The means for transmitting heat through said balloon may comprise one or more electrically heated wires in said tube for heating a liquid within said balloon, or laser wavelength produced by one or more optical fibers within said tube for heating a liquid within said balloon. The liquid may further comprise a biocompatible component that absorbs the specific laser wavelength used so that the liquid heats rapidly. Specifically, said means for transmitting heat through said balloon by heating a liquid may comprise an optical fiber and one or more metallic elements within the balloon; said optical fiber heating the one or more metallic elements within the balloon, said heat produced being transferred to the liquid within said balloon, said heated liquid transferring heat to the region of the artery.

One the other hand, said means for transmitting heat may also comprise a chemical exothermic reaction, preferably including the dissolution of magnesium sulfate powder or the interaction of silver chloride and sodium hydroxide solutions.

Preferably, said means for transmitting heat comprises means for heating the balloon surface directly, said balloon then heating the surrounding artery. Said means for heating the balloon surface directly may comprise a thermal conductive material within the membrane of the balloon, which material may be a fine wire meshwork that can be heated electrically, or a meshwork of optical fibers that diffuse light along their entire length, said fibers being coupled to the output of a laser and further comprising one or more components within the balloon that absorb the wavelength of the specific laser used to heat the balloon material, or it may comprise a wire meshwork within the balloon material that can be heated by the output of a laser, or a biocompatible gas in the interior of said balloon, wherein energy is delivered to the interior of the balloon and absorbed by the balloon material or by another absorbing surface provided within the body of the balloon.

Specifically, said means for transmitting heat may comprise: a laser; and a dye, said dye being capable of absorbing the specific wavelength of said laser used; said dye being applied to the plaque and any injured portion of the arterial wall; said laser and said dye heating the tissue directly without first heating either the fluid within the balloon or the balloon material.

In another embodiment of the angioplasty device of the invention, said means for transmitting heat at the artery region comprises: an optical fiber, part of said optical fiber being positioned within said balloon, said optical fiber including a light disseminating termination at the interior of the balloon structure; means for injecting laser light of a specific wavelength into one end of said fiber; and a transparent fluid within said balloon; wherein said balloon is transparent to radiation of the wavelength utilized. Said transparent fluid within said balloon may further comprise biocompatible pigments that absorb the specific laser wavelength used, and preferably it is water.

Although the most important application of this novel device is to improve PTCA of coronary arteries, this device can also be applied in atherosclerotic arteries located elsewhere, such as the renal, iliac, femoral and popliteal arteries.

In addition, this device may be applied in carotid arteries, unlike conventional angioplasty, because thermal fusion of the disrupted arterial tissues prevents embolization of tissue fragments to the brain.

In a preferred embodiment, the balloon is filled with transparent liquid and the balloon itself is transparent. An optical fiber is present within the catheter which has a light disseminating termination within the balloon structure. When this device is used, laser light may be injected into one end of the fiber and be disseminated by the disseminating means at the end of the fiber within the balloon such that the light travels virtually unaffected through the liquid in the balloon and through the balloon itself to the surrounding tissue which contains a certain amount of blood. When utilizing a neodymium : YAG laser at 1.06 microns, it has been found that this radiation while weakly absorbed by the surrounding tissue is strongly absorbed by the blood which causes the required fusion.

In studies of fresh postmortem human atherosclerotic arteries, filled with heparinized blood, it was found that fusion of plaque fragments and plaque-media separations could be easily achieved with this device without vaporizing any portion of the plaque or normal arterial wall. Histologic examination of formalin-fixed arterial specimens treated with this device showed a coagulum at the junction between layers or fragments of tissue, while no overt evidence of damage to the normal arterial wall was found. Moreover, no tissue adhesion to the balloon material occurred, and no damage occurred to the balloon itself, which could be made from one of a variety of high temperature plastic materials, such as a silicone polymer (Silastic), Reynolds even cooking bags and plastic sheets of autoclave packaging material.

The thermal fusion of disrupted arterial tissues appeared to be facilitated not only by preferential absorption of the neodymium : YAG radiation by blood between separated tissue layers, but also by the increase in tissue pressure produced first by balloon inflation and second by tissue shrinking from heating, the latter effect very likely representing initial protein cross linking. When arterial tissue is heated to temperatures greater than 70 degrees C, tissue shrinkage will ordinarily occur as a result of cross linking of proteins. However, when using the present device, the fixed volume of fluid within the balloon prevents the lumen cross section from decreasing in size during thermal fusion of tissues. Since the balloon is deflated after completion of thermal fusion, the lumen cross section following balloon deflation is not significantly smaller than the inflated balloon.

The following list describes alternative means which can be used to heat the tissues.

The first means is one for heating a liquid within the balloon of the angioplasty catheter. Any biocompatible liquid can be used within the balloon, typically a normal saline/radiographic contrast medium mixture, which is heated with one or more electrically heated wires. Alternatively the liquid is heated by laser energy delivered from one or more optical fibers. In the latter embodiment a biocompatible component of the liquid absorbs the specific laser wavelength used. For example, hemoglobin dissolved or suspended in the liquid strongly absorbs the energy of an argon-ion laser. As a result the liquid heats rapidly. Other examples of biocompatible pigments, which can be used in a similar manner, include Evan's Blue, methylene blue and Congo red. If water is used as the absorber, no visible pigments are required. Many different lasers, such as the $CO_2$ laser operating at the 10.6 micron wavelength, can be used to heat water efficiently.

In another embodiment, laser energy transmitted by an optical fiber is used to heat one or more metallic elements, such as wires, within or near the balloon.

In another embodiment, the liquid is heated by a chemical exothermic reaction. Both the reactants and the products are biocompatible. Examples include the dissolution of magnesium sulfate powder or the interaction of silver chloride and sodium hydroxide solutions. Means for injecting the individual components required for the exothermic reaction into the balloon cavity either simultaneously or sequentially are provided.

In another embodiment, means for heating the balloon directly are provided. As an example, thermal conductive material within the membrane of the balloon is heated directly. In one embodiment, a fine wire meshwork within the substance of the balloon is heated electrically. Alternatively, a meshwork of optical fibers, each of which is "lossy", i.e., each fiber diffuses light along its length, is coupled to the output of a laser. One or more components of the material within the balloon absorb the wavelength of the specific laser used and results in heating the balloon material. In another embodiment, laser energy is used to heat a wire meshwork within the balloon material. In yet another embodiment, the laser energy is delivered to the cavity of the balloon, with the balloon filled with a biocompatible gas such as $CO_2$, the energy being absorbed only by the balloon material, or other absorber(s) provided within the interior of the balloon.

In a still further embodiment, means for heating the tissues directly are provided. The plaque, the normal arterial wall, blood within the arterial wall, or any or all of these tissues are heated directly by these means without first heating either the liquid within the balloon or the balloon material. For example, the plaque and any injured portion of the arterial wall, when routine PTCA is performed prior to application of thermal energy during subsequent balloon inflation, may be stained with a dye, such as Evan's Blue or methylene blue. The energy of a Krypton or an argon-ion laser, among other lasers, is absorbed by the pigmented tissue and not by a translucent liquid or gas-filled balloon. Hemoglobin within blood, which has entered the arterial wall or spaces created by fractures from PTCA performed in a routine manner, acts as a natural pigment and is selectively heated by the output of any of a variety of lasers, such as an argon-ion laser.

An additional and optional element of the subject device is a sound transducer which in conjunction with the laser irradiation is a means for sensing intra-arterial sound produced by the laser irradiation heating of the tissue. In one embodiment, a catheter sound transducer similar to a high fidelity end-tipped manometer used to measure intravascular blood pressure is positioned within the outer catheter guide sheath near the balloon.

When using all of the foregoing embodiments of the present device energy in the form of laser radiation or heat is delivered to the entire circumference of the arterial cross section homogeneously along, typically, a 5 to 10 mm length of arterial wall. However, another embodiment of the present device comprises means for applying the laser energy or heat only to discrete locations around the arterial cross section and/or along the length of the artery. One or more of these said locations may be treated either in a random manner or on the basis of which portion of the artery would most likely benefit from the treatment as ascertained from the angiographic appearance of the arterial lumen. When laser radiation is used as described in one embodiment, the diffusing tip is constructed such that radially directed emission of the radiation occurs only at discrete locations along the diffusing tip. In one such embodiment, the surface of the diffusing tip, except for the discrete location, around the tip which permit emission of laser radiation, is coated with a highly reflective material such as gold.

In another embodiment, the diffusing tip consists of the termination of multiple optical fibers at different locations within the balloon. Each fiber, by a variety of means, such as an angled optical cleave, a miniature side-directing lens, or a prism, would emit laser radiation towards a discrete location within the arterial wall. In one embodiment, means for controlling the laser transmission through each fiber independently from laser transmission through the other fibers are provided.

## BRIEF DESCRIPTION OF THE DRAWINGS

These and other features of the subject invention will be better understood in connection with the detailed description taken in connection with the drawings of which :

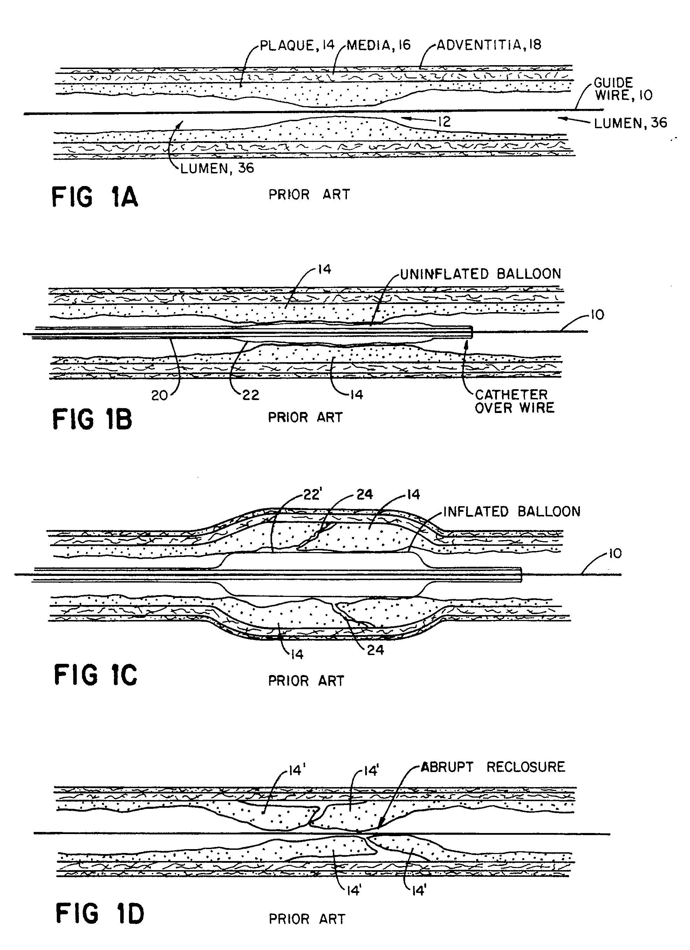

Figs. 1A-1D are diagrammatic illustrations of a percutaneous transluminal coronary angioplasty procedure in which plaque formed within the coronary artery is fractured initially by the inflation of a prior art balloon catheter and in which after deflation of the balloon and removal of the catheter abrupt reclosure can occur with the flaps of fragmented plaque occluding the coronary artery ;

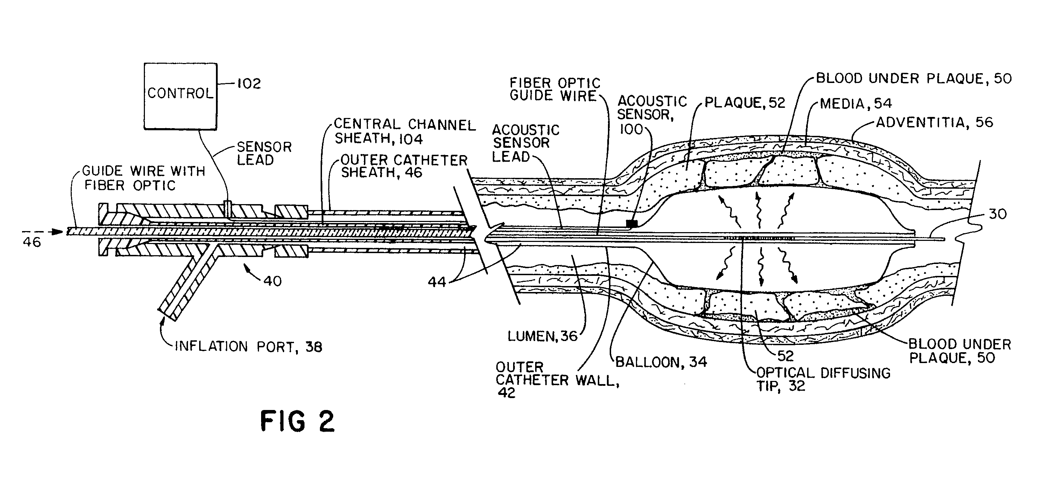

Fig. 2 is a cross-sectional and diagrammatic illustration of one embodiment of a balloon catheter of the invention in which the area immediately surrounding the inflated balloon is fused due to laser radiation which is transmitted through the fluid of the balloon and through the transparent balloon wall to blood within the wall of the coronary artery segment being treated, which heated blood causes the welding of adjacent, disrupted layers throughout the plaque, media and adventitia, resulting in a smooth channel once the balloon is deflated;

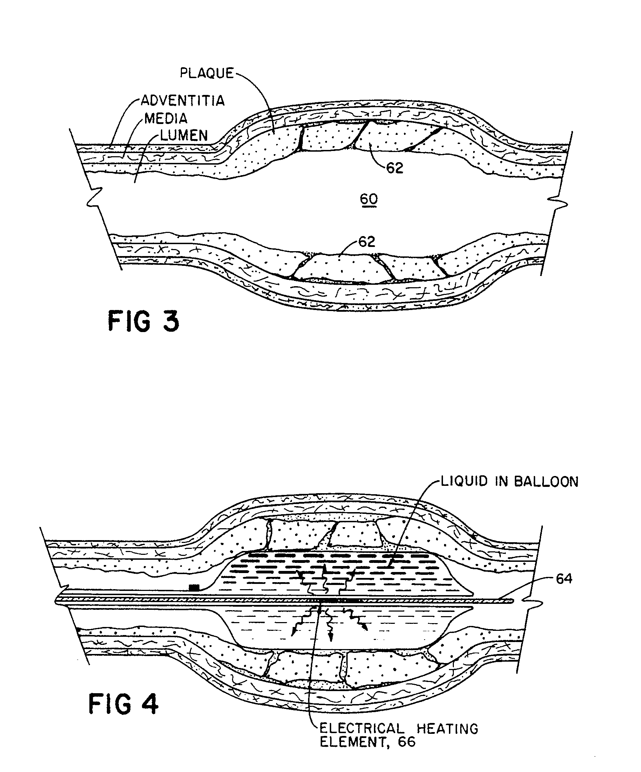

Fig. 3 is a cross-sectional and diagrammatic illustration of the result of utilizing the balloon catheter of Fig. 2 illustrating the channel formed by the fusion which takes place due to fusing produced by heat in the vicinity of the balloon wall;

Fig. 4 is a cross-sectional and diagrammatic illustration of an alternative embodiment of the balloon catheter of the invention in which liquid within the balloon is electrically heated, which heat is then utilized in the fusion process to produce the result illustrated in Fig. 3;

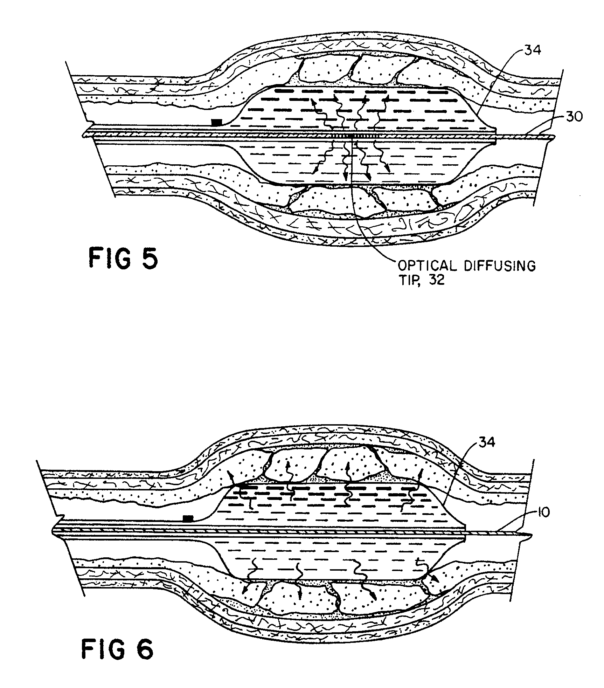

Fig. 5 is a cross-sectional and diagrammatic illustration of a still further embodiment of the balloon catheter of the invention in which liquid within the balloon is heated by laser radiation emanating from an optical diffusing tip thereby to heat the liquid within the balloon for the production of fusion and the result of Fig. 3;

Fig. 6 is a cross-sectional and diagrammatic illustration of a still further embodiment of the balloon catheter of the invention in which heat within the balloon is produced by an exothermic chemical reaction of chemical components pumped into the balloon, with the resulting heat radiating outwardly to produce the fusion result of Fig. 3;

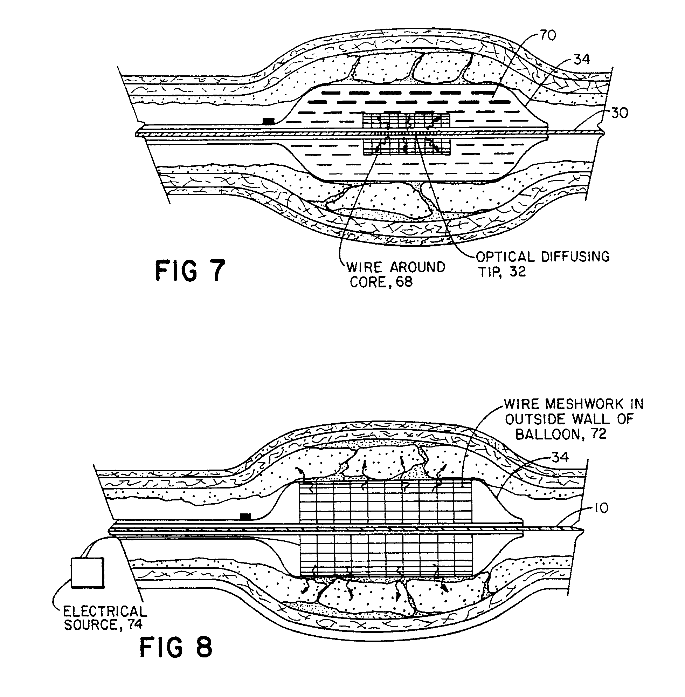

Fig. 7 is a cross-sectional and diagrammatic illustration of an alternative embodiment of the balloon catheter of the invention in which a wire core is provided adjacent an optical diffusing tip with the laser radiation being tuned to a wavelength which heats the wires which then heats the liquid within the balloon which in turn heats the surrounding material to provide the fusion result of Fig. 3;

Fig. 8 is a cross-sectional and diagrammatic illustration of another embodiment of the balloon catheter of the invention in which the balloon wall is provided with a network of electrical wires and in which the balloon is heated by virtue of the application of electrical energy to the wires which heats the area surrounding the balloon thereby to provide the fusion result of Fig. 3;

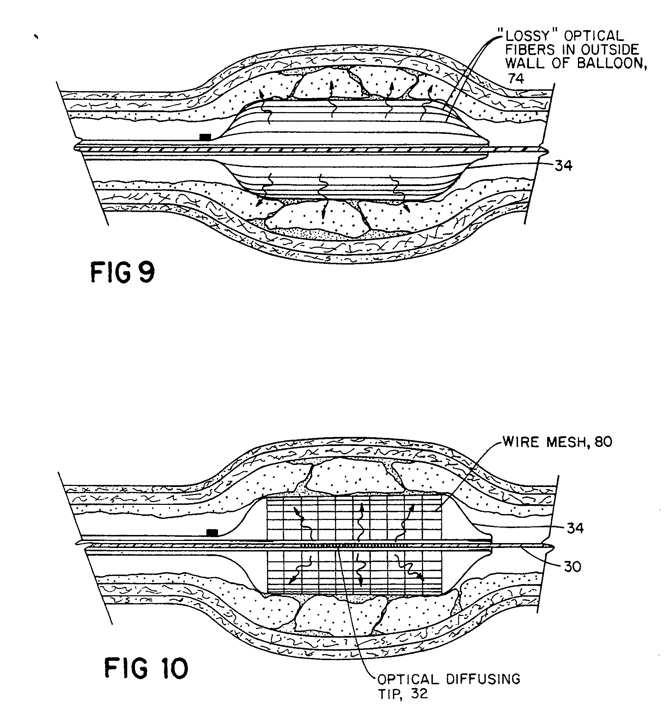

Fig. 9 is a cross-sectional and diagrammatic illustration of another embodiment of the balloon catheter of the invention in which the balloon is provided with a number of "lossy" optical fibers along or adjacent the outside wall of the balloon and in which laser radiation is transmitted through these "lossy" optical fibers, thereby to heat adjacent components either within the balloon or within the coronary artery to provide the fusion result of Fig. 3; and

Fig. 10 is a cross-sectional and diagrammatic illustration of another embodiment of the balloon catheter of the invention in which a wire mesh is provided at or adjacent the walls of the balloon and in which an optical diffusing tip is provided with radiation of an energy which is absorbed by the wires thereby to heat the wires and thus the surrounding coronary wall tissue to provide the fusion result of Fig. 3.

## DETAILED DESCRIPTION

As mentioned hereinbefore, during percutaneous transluminal coronary angioplasty or indeed any type of balloon angioplasty and referring now to Figs. 1A-1D, in the prior art a guide wire 10 is inserted through the artery and through the region 12 which is occluded primarily by plaque 14 which is surrounded by media 16 and by adventitia 18. It is recognized that it is the plaque which forms the occlusion. A prior art balloon catheter 20 is provided around guide wire 10 which includes a deflated balloon section 22 which surrounds the wire.

This balloon section is placed adjacent to plaque 14 and as illustrated in Fig. 1C is inflated as illustrated at 22' which opens up the artery while at the same time providing fissures or dissected planes of tissue 24. As illustrated in Fig. 1D, with the catheter removed, the plaque 14 can collapse into the center of the artery as illustrated by flap portions 14' which results in an abrupt reclosure of the artery that can result in an acute myocardial infarction.

Referring to Fig. 2 in a preferred embodiment, the usual guide wire is replaced with an optical fiber 30 which has an optical diffusion area or tip 32 midway in the region of an inflated balloon 34 such that, when the catheter is inserted around the optical fiber in lumen 36 and expanded by virtue of providing a transparent fluid through inflation port 38 in termination apparatus generally indicated at 40, the fluid passes through the catheter wall 42 by virtue of a channel 44 in the outer catheter sheath 46 such that balloon 34 is initially inflated. After inflation, laser radiation indicated by dotted arrow 46 is introduced into the optical fiber 30 and is transmitted to the optical diffusion tip 32 where the laser light is diffused and impinges upon blood 50 which usually exists within the arterial wall after fracture or dissection of the plaque has occurred following conventional balloon inflation, with the plaque 52 being held in place upon completion of the fusion process in which disrupted arterial wall elements, media 54 and adventitia 56, are raised to the fusion temperature. The fluid utilized for inflation of the balloon may be contrast medium or crystalloids such as normal saline or 5% dextrose in water and is relatively transparent to the radiation which, in the embodiment shown, is on the order of 1.06 microns which is highly absorbed by the blood. The blood within the arterial wall is therefore heated and this heat radiates to fuse the area immediately surrounding balloon 34 such that as illustrated in Fig. 3 upon removal of the catheter a smooth channel 60 exists with relatively smooth walls 62 and with the surrounding tissue having been fused to prevent the aforementioned reclosure.

The diffusing tip is centrally located within balloon 34 and the length of said tip is typically one half or less the length of the balloon. Thus, tissues at the proximal and distal ends of balloon 34 are not heated, and no thermal injury occurs to blood within lumen 36.

It will be appreciated that a diffusing tip or area along an otpical fiber may be provided simply by removing the cladding and abraiding the fiber core surface such that the fiber surface is roughened. Alternatively the diffusing tip 32 can be made of a material which effectively scatters the laser energy and the diffusing tip would then be coupled to the output end of the optical fiber. In another embodiment, the optical fiber lies within channel 44 and terminates directly within the balloon. The diffusing tip 32 in this latter embodiment could be similar in design to diffusing tips of the aforementioned embodiments, but could also be comprised of the fluid used to inflate balloon 34. Examples of biocompatible fluids which would be effective scattering media for laser radiation include dilute lipid emulsions and perfluoro-chemical emulsions. In other embodiments, the optical fiber would lie within the wall of either the outer catheter sheath 46 or the central channel sheath 104.In yet another embodiment, the central channel sheath 104 is comprised of a hollow optical fiber with a concentric core-cladding arrangement which would terminate in a diffusing tip. Laser energy would then be transmitted within the core of the optical fiber, and the hollow interior of the fiber would allow coaxial placement of a guide wire through the central channel sheath during the laser energy transmission period.

It will be appreciated that the balloon is in the form of a doughnut in cross section which is in essence slipped over the guide wire or optical fiber and inflated when the balloon is in proper position.

Referring to Fig. 4, instead of the utilization of fiber optic transmission of energy to the balloon area, electrical conductors 64 may be utilized as a guide wire in which an electrical heating element 66 is driven from a power source (not shown in this figure) to provide heat to fluid within the balloon which then heats the surrounding tissue to provide the aforementioned fusion. The liquid in this case may be crystalloids or a liquid with a high boiling point such as Dow Corning 550 silicone diluent which transmits the heat from the heating element to the walls of the balloon and then to the tissue immediately surrounding these walls.

Referring to Fig. 5, a similar result may be obtained by utilizing the optical fiber 30 and optical diffusing tip 32 in which, rather than having a transparent fluid within balloon 34, an absorbing fluid is utilized which absorbs laser energy, normally in the 0.3-10.6 micron range, with the liquid within balloon 34 being selected from the biocompatible group including water, hemoglobin and related pigments such as bilirubin and porohyrins, Congo red, Evan's Blue and methylene blue, and other materials absorbing in this range. The result is similar in that heat is directed to the tissue immediately surrounding the surface of the balloon which causes the aforementioned fusion.

Referring to Fig. 6 and a still further embodiment, heat may be generated within balloon 34 which has been inserted around a conventional guide wire 10 in which an exothermic reaction is permitted to occur once the balloon has been inflated to release energy into the surrounding tissue thereby, again, to provide for fusion of the tissue surrounding the inflated balloon.

Referring now to Fig. 7, energy may be introduced to the balloon 34 by virtue of the utilization of optical fiber 30 with diffusing tip 32 which is surrounded by a wire core 68 which may be expandable, adapted to absorb the radiation from the diffusing tip. In this embodiment, the wire made of an absorbing material such as stainless steel, platinum, gold, silver or tungsten and the radiation utilized to heat the wire core is on the order of 0.3 to 10.6 microns in one embodiment. In this embodiment the wire core heat liquid or fluid 70 within the balloon which again transmits heat to the required area for the required fusion.

In another embodiment, the optical fiber terminates in a sleeve or cap of an absorbing material which absorbs the laser energy and heats the liquid.

Referring to Fig. 8, ballon 34 itself may be provided with a meshwork of electrical wires 72 which are driven by an electrical source 74 which serves to heat the exterior of the balloon once it has been inserted around conventional guide wire 10. In this embodiment, it is the surface of the balloon which is heated directly by the use of electrical energy to produce the aforementioned fusion.

In a still further alternative embodiment and referring now to Fig. 9, the surface of balloon 34 may be provided with "lossy" optical fibers 76 which are driven by laser radiation and which radiate energy in all directions outwardly to the tissue surrounding balloon 34. In such a case, 1.06 micron radiation may be utilized which in turn heats the blood surrounding the inflated balloon thereby causing the aforementioned fusion result of Fig. 3.

In a still further embodiment and referring now to Fig. 10, the exterior or interior of the balloon wall may be provided with a wire mesh 80 which, as in the Fig. 7 embodiment, is heated through diffused radiation from diffusing tip 32 which is located along optical fiber 30. In this embodiment optical radiation is absorbed in the wire mesh which heats up and causes the aforementioned fusion.

The amount of energy delivered to the wall of the balloon and the surrounding tissue varies from 50 to 1000 joules depending upon the size of the artery and the method of introducing energy into the region. Typically, in terms of 5 to 20 watts of neodymium: YAG laser power is necessary for 10 to 35 seconds for a balloon having a longitudinal extent of 2 cm, a diameter of 3 mm when the balloon is inflated, and a diffusing tip of one centimeter for irradiating an arterial segment one centimeter in length. When only discrete region(s) within the arterial wall adjacent to the inflated balloon are heated, significantly less energy is necessary to achieve tissue fusion.

Moreover as mentioned above, a special dye may be utilized such that the blood and/or plaque tissue more strongly absorbs light of a given wavelength thereby increasing the chance of fusion while at the same time decreasing the overall time for the procedure.

Referring back now to Fig. 2, an acoustic sensor 100 may be placed adjacent to balloon 34 for the purpose of sensing the sounds produced by the laser irradiation heating of the tissue. A catheter transducer similar to the transducer incorporated in an end-tipped manometer catheter for measurement of intravascular pressure is positioned within the outer catheter near the balloon site. If the laser radiation incident on the plaque through the balloon wall is intense enough to cause vaporization, a high frequency acoustic signal is generated which is picked up by the sound transducer and relayed to a control unit 102 which is utilized either to indicate that vaporization has taken place and/or to automatically terminate the emission of laser radiation or other heating source power delivery.

## Claims

1. An angioplasty device comprising a catheter suitable for insertion into an artery and having: a tube defining a lumen; an inflatable balloon (34) secured to said tube for inflation from a remote source of fluid; and means (38, 40, 44) for inflating said balloon (34); said balloon (34) being configured so that said tube may be navigated through the artery when deflated and allow blood to flow; and the means (38, 40, 44) for inflating said balloon (34) being configured so that they may inflate said balloon (34) with fluid when said balloon (34) is in an area of stenosis of the artery to apply pressure to the area of stenosis of said artery to widen the inner diameter of the artery at that area; characterized in that the device comprises means (30, 32; 64, 66; 30, 32, 68, 70; 72, 74; 76; 80) for transmitting heat through the balloon (34).

2. The angioplasty device of claim 1 wherein said means for transmitting heat through said balloon (34) comprises one or more electrically heated wires (64, 66) in said tube for heating a liquid within said balloon (34).

3. The angioplasty device of claim 1 wherein said means for transmitting heat through said balloon (34) comprises one or more optical fibers (30) within said tube for heating a liquid within said balloon (34) by transmission of laser radiation along the optical fibers.

4. The angioplasty device of claim 3 wherein said liquid further comprises a biocompatible component that absorbs the specific laser wavelength used so that the liquid heats rapidly.

5. The angioplasty device of claim 1 wherein said means for transmitting heat through said balloon (34) by heating a liquid (70) comprises an optical fiber (30) and one or more metallic elements (68) within the balloon; said optical fiber (30) being adapted to heat the one or more metallic elements (68) within the balloon (34), said heat produced being transferred to the liquid (70) within said balloon (34), said heated liquid (70) transferring heat to the region of the artery.

6. The angioplasty device of claim 1 wherein said means for transmitting heat comprises means for generating a chemical exothermic reaction.

7. The angioplasty device of claim 6 wherein said chemical exothermic reaction includes the dissolution of magnesium sulfate powder.

8. The angioplasty device of claim 6 wherein said chemical exothermic reaction includes the interaction of silver chloride and sodium hydroxide solutions.

9. The angioplasty device of claim 1 wherein said means for transmitting heat comprises means (72, 74) for heating the balloon (34) surface directly, said balloon (34) then heating the surrounding artery.

10. The angioplasty device of claim 9 wherein said means (72, 74) for heating the balloon surface directly comprises a thermal conductive material within the membrane of the balloon.

11. The angioplasty device of claim 10 wherein said thermal conductive material is a fine wire meshwork (72) that can be heated electrically.

12. The angioplasty device of claim 10 wherein said thermal conductive material is a meshwork of optical fibers (76) that diffuse light along their entire length, said fibers (76) being coupled to the output of a laser and further comprising one or more components within the balloon (34) that absorb the wavelength of the specific laser used to heat the balloon (34) material.

13. The angioplasty device of claim 10 wherein said thermal conductive material comprises a wire meshwork (80) within the balloon (34) material that can be heated by the output of a laser.

14. The angioplasty device of claim 10 wherein said means for heating the balloon (34) surface directly comprises a biocompatible gas in the interior of said balloon (34), wherein energy may be delivered to the interior of the balloon (34) and absorbed by the balloon (34) material or by another absorbing surface provided within the body of the balloon (34).

15. The angioplasty device of claim 1 wherein said means for transmitting heat comprises: a laser; and a dye, said dye being capable of absorbing the specific wavelength of said laser used; said dye being suitable for application to the plaque and any injured portion of the arterial wall; said laser and said dye being adapted to heat the tissue directly without first heating either the fluid (70) within the balloon (34) or the balloon (34) material.

16. The angioplasty device of claim 1 wherein said means for transmitting heat at the artery region comprises: an optical fiber (30), part of said optical fiber (30) being positioned within said balloon, said optical fiber including a light disseminating termination at the interior of the balloon (34) structure; means (32) for injecting laser light of a specific wavelength into one end of said fiber; and a transparent fluid (70) within said balloon; wherein said balloon (34) is transparent to radiation of the wavelength utilized.

17. The angioplasty device of claim 16 wherein said transparent fluid (70) further comprises biocompatible pigments that absorb the specific laser wavelength used.

18. The angioplasty device of claim 16 wherein said transparent fluid (70) is water.

**Revendications**

1. Dispositif pour angioplastie comprenant un cathéter convenant à l'insertion dans une artère et ayant :
   un tube définissant un passage ;
   un ballon gonflable (34) fixé audit tube pour être gonflé depuis une source éloignée de fluide ; et

des moyens (38, 40, 44) pour gonfler ledit ballon (34) ;

ledit ballon (34) étant configuré de telle sorte que ledit tube peut être conduit au travers de l'artère lorsqu'il est dégonflé et permettre au sang de couler ; et

les moyens (38, 40, 44) pour le gonflage du ballon (34) étant configurés de telle sorte qu'ils peuvent gonfler ledit ballon (34) à l'aide d'un fluide lorsque ledit ballon (34) est dans une zone de sténose de ladite artère pour appliquer une pression à la zone de sténose de ladite artère et agrandir le diamètre interne de l'artère dans cette zone ; caractérisé en ce que ledit dispositif comprend des moyens (30, 32 ; 64, 66 ; 30, 32, 68, 70 ; 72, 74 ; 76, 80) pour transmettre de la chaleur par l'intermédiaire du ballon (34).

2. Dispositif pour angioplastie selon la revendication 1, dans lequel lesdits moyens de transmission de chaleur par l'intermédiaire dudit ballon (34) comprennent un ou plusieurs fils (64, 66) chauffés électriquement dans ledit tube pour chauffer un liquide se trouvant dans ledit ballon (34).

3. Dispositif pour angioplastie selon la revendication 1, dans lequel lesdits moyens de transmission de chaleur par l'intermédiaire dudit ballon (34) comprennent une ou plusieurs fibres optiques (30) dans ledit tube pour chauffer un liquide se trouvant dans ledit ballon (34) par transmission d'un rayonnement laser le long des fibres optiques.

4. Dispositif pour angioplastie selon la revendication 3, dans lequel ledit liquide renferme, en outre, un composant biocompatible qui absorbe la longueur d'onde laser spécifique utilisée, de telle sorte que le liquide s'échauffe rapidement.

5. Dispositif pour angioplastie selon la revendication 1, dans lequel lesdits moyens de transmission de la chaleur par l'intermédiaire dudit ballon (34) par chauffage d'un liquide (70) comprennent une fibre optique (30) et un ou plusieurs éléments métalliques (68) à l'intérieur dudit ballon ; ladite fibre optique (30) étant adaptée à chauffer le ou les éléments métalliques (68) dans le ballon (34), la chaleur ainsi produite étant transférée au liquide (70) à l'intérieur dudit ballon (34), le liquide chauffé (70) transférant de la chaleur à la région de l'artère.

6. Dispositif pour angioplastie selon la revendication 1, dans lequel lesdits moyens de transmission de la chaleur comprennent des moyens pour générer une réaction chimique exothermique.

7. Dispositif pour angioplastie selon la revendication 6, dans lequel ladite réaction chimique exothermique comprend la dissolution de poudre de sulfate de magnésium.

8. Dispositif pour angioplastie selon la revendication 6, dans lequel ladite réaction chimique exothermique comprend l'interréaction de solutions de chlorure d'argent et d'hydroxyde de sodium.

9. Dispositif pour angioplastie selon la revendication 1, dans lequel lesdits moyens de transmission de la chaleur comprennent des moyens (72, 74) pour chauffer la surface du ballon (34) directement, ledit ballon (34) chauffant ensuite l'artère environnante.

10. Dispositif pour angioplastie selon la revendication 9, dans lequel lesdits moyens (72, 74) pour chauffer directement la surface du ballon comprennent un matériau conducteur thermique à l'intérieur de la membrane du ballon.

11. Dispositif pour angioplastie selon la revendication 10, dans lequel ledit matériau conducteur thermique est un fin réseau de fils (72) qui peut être chauffé électriquement.

12. Dispositif pour angioplastie selon la revendication 10, dans lequel ledit matériau conducteur thermique est un réseau de fibres optiques (76) qui diffusent de la lumière sur toute leur longueur, lesdites fibres (76) étant associées à la sortie d'un laser et comprenant, en outre, un ou plusieurs composants dans le ballon (34) qui absorbent la longueur d'onde du laser spécifique utilisé pour chauffer le matériau du ballon (34).

13. Dispositif pour angioplastie selon la revendication 10, dans lequel ledit matériau conducteur thermique comprend un réseau de fils (80) dans le matériau du ballon (34) qui peuvent être chauffés par la sortie d'un laser.

14. Dispositif pour angioplastie selon la revendication 10, dans lequel lesdits moyens de chauffage direct de la surface du ballon (34) comprennent un gaz biocompatible à l'intérieur dudit ballon (34), de l'énergie pouvant être délivrée à l'intérieur dudit ballon (34) et absorbée par le matériau dudit ballon (34) ou par une autre surface absorbante prévue à l'intérieur du corps du ballon (34).

15. Dispositif pour angioplastie selon la revendication 1, dans lequel lesdits moyens de transmission de la chaleur comprennent : un laser, un colorant, ledit colorant étant capable d'absorber la longueur d'onde spécifique dudit laser utilisé ; ledit colorant convenant pour l'application à la plaque ou à toute portion endommagée de la paroi de l'artère ; ledit laser et ledit colorant étant adaptés à chauffer le tissu directement sans chauffer tout d'abord ni le fluide (70) dans le ballon (34), ni le matériau du ballon (34).

16. Dispositif pour angioplastie selon la revendication 1, dans lequel lesdits moyens de transmission de la chaleur à la région de l'artère comprennent : une fibre optique (30), une partie de ladite fibre optique (30) étant disposée à l'intérieur du ballon, ladite fibre optique comprenant une extrémité disséminatrice de lumière à l'intérieur de la structure du ballon (34) ; des moyens (32) pour injecter de la lumière laser à une longueur d'onde spécifique dans une extrémité de ladite fibre ; et un fluide transparent (70) à l'intérieur dudit ballon ; ledit ballon (34) étant transparent au rayonnement de la longueur d'onde utilisée.

17. Dispositif pour angioplastie selon la revendication 16, dans lequel ledit fluide transparent (70) comprend, en outre, des pigments biocompatibles qui absorbent la longueur d'onde spécifique du laser utilisé.

18. Dispositif pour angioplastie selon la revendication 16, dans lequel ledit fluide transparent (70) est de l'eau.

**Patentansprüche**

1. Angioplastievorrichtung, die einen Katheter aufweist, der zum Einführen in eine Arterie geeignet ist und aufweist:
ein ein Lumen definierendes Rohr;
einen aufblasbaren Ballon (34), der zum Aufblasen von einer entfernten Fluidquelle an dem Rohr befestigt ist; und eine Einrichtung (38, 40, 44) zum Aufblasen des Ballons (34); wobei der Ballon (34) so ausgebildet ist, daß das Rohr bei entleertem Ballon durch die Arterie geführt werden kann und das Strömen von Blut zuläßt; und
wobei die Einrichtung (38, 40, 44) zum Aufblasen des Ballons (34) so ausgebildet ist, daß sie diesen mit Fluid aufblasen kann, wenn er sich in einem Stenosebereich der Arterie befindet, um auf den Stenosebereich der Arterie Druck aufzubringen und dadurch den Innendurchmesser der Arterie in diesem Bereich zu erweitern;
dadurch gekennzeichnet, daß die Vorrichtung eine Einrichtung (30, 32; 64, 66; 30, 32, 68; 70; 72, 74; 76; 80) zum Übertragen von Wärme durch den Ballon (34) aufweist.

2. Angioplastievorrichtung nach Anspruch 1, wobei die Einrichtung zum Übertragen von Wärme durch den Ballon (34) einen oder mehrere elektrisch geheizte Drähte (64, 66) in dem Rohr aufweist, die eine Flüssigkeit in dem Ballon (34) erwärmen.

3. Angioplastievorrichtung nach Anspruch 1, wobei die Einrichtung zum Übertragen von Wärme durch den Ballon (34) einen oder mehrere Lichtwellenleiter (30) in dem Rohr aufweist, die durch Übertragung von Laserstrahlung entlang den Lichtwellenleitern eine Flüssigkeit in dem Ballon (34) erwärmen.

4. Angioplastievorrichtung nach Anspruch 3, wobei die Flüssigkeit ferner eine biokompatible Komponente aufweist, die die angewandte spezielle Laserwellenlänge absorbiert, so daß die Flüssigkeit rasch erwärmt wird.

5. Angioplastievorrichtung nach Anspruch 1, wobei die Einrichtung zum Übertragen von Wärme durch den Ballon (34) durch Erwärmen einer Flüssigkeit (70) einen Lichtwellenleiter (30) und ein oder mehrere Metallelemente (68) in dem Ballon aufweist; wobei der Lichtwellenleiter (30) so ausgebildet ist, daß er die ein oder mehreren Metallelemente (68) in dem Ballon (34) erwärmt, wobei die erzeugte Wärme auf die Flüssigkeit (70) in dem Ballon (34) übertragen wird und die erwärmte Flüssigkeit (70) Wärme auf den Bereich der Arterie überträgt.

6. Angioplastievorrichtung nach Anspruch 1, wobei die Einrichtung zum Übertragen von Wärme eine Einrichtung zur Erzeugung einer chemischen exothermen Reaktion aufweist.

7. Angioplastievorrichtung nach Anspruch 6, wobei die chemische exotherme Reaktion das Auflösen von Magnesiumsulfatpulver umfaßt.

8. Angioplastievorrichtung nach Anspruch 6, wobei die chemische exotherme Reaktion die Wechselwirkung zwischen Silberchlorid- und Natriumhydroxidlösungen umfaßt.

9. Angioplastievorrichtung nach Anspruch 1, wobei die Einrichtung zum Übertragen von Wärme eine Einrichtung (72, 74) aufweist, die die Oberfläche des Ballons (34) direkt erwärmt, woraufhin der Ballon (34) die umgebende Arterie erwärmt.

10. Angioplastievorrichtung nach Anspruch 9, wobei die Einrichtung (72, 74) zum direkten Erwärmen der Ballonoberfläche in der Ballonmembran ein wärmeleitfähiges Material aufweist.

11. Angioplastievorrichtung nach Anspruch 10, wobei das wärmeleitfähige Material ein elektrisch heizbares Haardrahtnetzwerk (72) ist.

12. Angioplastievorrichtung nach Anspruch 10, wobei das wärmeleitfähige Material ein Netzwerk von Lichtwellenleitern (76) ist, die Licht entlang ihrer Gesamtlänge streuen, wobei die Lichtwellenleiter (76) mit dem Ausgang eines Lasers gekoppelt sind, und ferner umfassend eine oder mehrere Komponenten in dem Ballon (34), die die Wellenlänge eines angewandten speziellen Lasers absorbieren und das Material des Ballons (34) erwärmen.

13. Angioplastievorrichtung nach Anspruch 19, wobei das wärmeleitfähige Material ein Drahtnetzwerk (80) in dem Material des Ballons (34) aufweist, das von der Ausgangsenergie eines Lasers erwärmbar ist.

14. Angioplastievorrichtung nach Anspruch 10, wobei die Einrichtung zum direkten Erwärmen der Oberfläche des Ballons (34) im Inneren des Ballons (34) ein biokompatibles Gas aufweist, wobei dem Inneren des Ballons (34) Energie zuführbar ist, die von dem Material des Ballons (34) oder einer anderen in dem Körper des Ballons (34) vorgesehenen Absorptionsfläche absorbierbar ist.

15. Angioplastievorrichtung nach Anspruch 1, wobei die Einrichtung zum Übertragen von Wärme aufweist: einen Laser; und einen Farbstoff, der die spezielle Wellenlänge des angewandten Lasers absorbieren kann; wobei der Farbstoff zum Aufbringen auf das Plaque und einen beschädigten Bereich der Arterienwand geeignet ist; wobei der Laser und der Farbstoff das Gewebe direkt erwärmen können, ohne zuerst entweder das Fluid (70) in dem Ballon (34) oder das Material des Ballons (34) zu erwärmen.

16. Angioplastievorrichtung nach Anspruch 1, wobei die Einrichtung zum Übertragen von Wärme in dem Arterienbereich aufweist: einen Lichtwellenleiter (30), wobei ein Teil dieses Lichtwellenleiters (30) in dem Ballon positioniert ist und der Lichtwellenleiter im Inneren des Aufbaus des Ballons (34) einen Lichtausbreitungsabschluß aufweist; eine Einrichtung (32) zum Injizieren von Laserlicht einer speziellen Wellenlänge in ein Ende des Lichtwellenleiters; und ein lichtdurchlässiges Fluid (70) in dem Ballon; wobei der Ballon (34) für Strahlung der angewandten Wellenlänge durchlässig ist.

17. Angioplastievorrichtung nach Anspruch 16, wobei das lichtdurchlässige Fluid (70) ferner biokompatible Pigmente aufweist, die die angewandte spezielle Laserwellenlänge absorbieren.

18. Angioplastievorrichtung nach Anspruch 16, wobei das lichtdurchlässige Fluid (70) Wasser ist.

FIG 1A    PRIOR ART

FIG 1B    PRIOR ART

FIG 1C    PRIOR ART

FIG 1D    PRIOR ART

FIG 2

PLAQUE
ADVENTITIA
MEDIA
LUMEN
62
60
62

FIG 3

LIQUID IN BALLOON
64
FIG 4
ELECTRICAL HEATING
ELEMENT, 66

FIG 5

OPTICAL DIFFUSING TIP, 32

34

30

FIG 6

34

10

FIG 7

WIRE AROUND CORE, 68

OPTICAL DIFFUSING TIP, 32

70
34
30

FIG 8

WIRE MESHWORK IN OUTSIDE WALL OF BALLOON, 72

ELECTRICAL SOURCE, 74

34
10

"LOSSY" OPTICAL
FIBERS IN OUTSIDE
WALL OF BALLOON,
74

34

**FIG 9**

WIRE MESH, 80

34

30

**FIG 10**

OPTICAL DIFFUSING
TIP, 32